# EUROPEAN PATENT SPECIFICATION

(11) **EP 2 010 040 B1**
(45) Date of publication and mention of the grant of the patent: **14.10.2015**
(21) Application number: 07718786.2
(22) Date of filing: 27.04.2007
(51) Int. Cl.: A61B 1/06, A61B 1/267, A61B 17/24

(54) **A LIGHT SOURCE APPARATUS**
LICHTQUELLENGERÄT
APPAREIL SOURCE DE LUMIÈRE

(30) Priority: 27.04.2006 AU 2006902163 P
(43) Date of publication of application: 07.01.2009
(73) Proprietor: Dunlop, Colin, East Ryde NSW 2113 (AU)
(72) Inventor: Dunlop, Colin, East Ryde NSW 2113 (AU)
(74) Representative: Dauncey, Mark Peter
(86) International application number: PCT/AU2007/000539
(87) International publication number: WO 2007/124536

(56) References cited:
- EP-A- 1 433 413
- EP-B1- 0 240 240
- WO-A1-96/20634
- WO-A2-2006/131770
- DE-A1- 4 243 790
- DE-C1- 3 815 251
- US-A- 4 273 112
- US-A- 4 607 623
- US-A- 5 879 304
- US-A1- 2003 193 314
- US-A1- 2005 187 434

## Description

The present invention relates to a lighting apparatus and, particularly, but not exclusively, to medical applications such as a light source for a hand held laryngoscope.

A laryngoscope is a device used to examine the larynx and its surrounding structures. A laryngoscope generally comprises a handle and a detachable blade. A source of illumination is positioned to direct light along the laryngoscope blade to illuminate the anatomical structures being viewed.

In use, the blade of the laryngoscope is inserted via the mouth into the pharynx and the light enables an inspection of the larynx and associated structures. Laryngoscopes are used for examining and diagnosing diseases of the mouth, pharynx and larynx including the vocal chords. Laryngoscopes are most commonly used for the purpose of inserting a tube through the glottis (intubating). During anaesthesia, a tube is passed via the mouth, through the larynx and into the trachea to maintain airway patency (endotracheal intubation) and permit administration of oxygen and inhalation anaesthetic. Laryngoscopes facilitate intubation by elevating the soft pallet, depressing the epiglottis and illuminating the larynx.

Laryngoscopes are used both in veterinary and human medicine. There are many different types of laryngoscope, and, particularly, many different shapes of laryngoscope blades.

A laryngoscope light source is conventionally powered by batteries usually mounted in the laryngoscope handle. Typically, the light source comprises a conventional incandescent bulb mounted about two thirds of the way down the laryngoscope blade with wiring tracking back to a mechanical contact at the proximal end of the blade. Installing wiring and connections in handles is difficult, time consuming and therefore costly. The blade contact makes a mechanical and electrical connection to a contact in the top of the handle (handle contact) when the blade is fitted to the handle and locked in its operational position. The handle contact is electrically connected to the power supply (usually conventional or rechargeable batteries). Generally the light source is arranged to be switched on when the blade is fitted to the handle and "locked" in its operational position and the mechanical and the electrical connection is mechanically actuated.

Conventional light bulbs placed in the blade suffer problems with contact caused by moisture in use, and often fail. Conventional incandescent bulbs operate to provide a high light output on a small DC drive voltage, so demand a high current, with a high heat output. These requirements limit the life of an incandescent element, resulting in frequent bulb failure. Conventional filament bulbs being hot to touch can also burn mucosa.

An alternative light source arrangement incorporates an optical fibre bundle running along the blade but still uses a conventional incandescent filament bulb mounted in the handle. The bulb transmits light to the optical fibre bundle to illuminate the tip of the laryngoscope blade. An electrical connection is mechanically activated between the blade and handle, and switches the bulb. In this alternative, it can be difficult to obtain access to the bulb in the handle to change the bulb.

In addition, in both the blade mounted and handle mounted bulb variations, the incandescent light bulb is not sealed, the switch is not sealed and there are therefore difficulties in sterilising either the blade or the handle or otherwise exposing the handle to fluid as it can affect the light source and electrical circuit powering the light source.

European Patent Application No. 03078524.0 (Western Sydney Area Health Service), discloses a laryngoscope which has an LED light source mounted with circuitry in a handle of the laryngoscope. The circuitry includes an induction coil mounted in the handle. The disclosure suggests the concept of utilising the coil or other component mounted in the laryngoscope blade, interacting with the coil in the handle mounted circuit to cause current to flow and power the LED, when the laryngoscope blade is attached to the laryngoscope handle.

In accordance with a first aspect, the present invention provides a light source apparatus for a laryngoscope, including a light source, an electrical circuit for providing power to the light source, a power source for providing power to the electrical circuit and a remotely operable switch for switching power to the light source, characterised in that the remotely operable switch is arranged to switch the electrical circuit from an off state in which no current flows in the circuit, to an on state in which current flows from the power source to the light source, to power the light source.

In an embodiment, the light source apparatus is arranged to fit within a laryngoscope handle.

The remotely operable switch is magnetically activated, such as a reed switch, for example. The magnetically activated switch is operated by a magnet mounted in a proximal part of a laryngoscope blade, so that when the laryngoscope blade is connected to the handle and locked in position, the magnetically activated switch is closed and power is provided to the light source.

In an embodiment the light source is a low power light emitting diode (LED) which provides cold light and has a long operational life compared to an incandescent bulb.

In an embodiment, the light source may be electronically current or voltage driven for constant spectral output and intensity.

In an embodiment the light apparatus is arranged to co-operate with an optical fibre bundle mounted in a laryngoscope blade, so that light is transmitted from the light source to the optical fibre bundle.

In an embodiment, the light source, switch and electrical circuit are mounted together in a sealed mounting. The entire sealed mounting assembly is arranged to fit into the handle of a laryngoscope. In an embodiment, the electrical circuit includes electrical contacts arranged for connection to a power source.

In the embodiment where the light source comprises an LED, and the switch, light source and contacts are mounted in a sealed mounting, the arrangement has the advantage that the light source lasts a long time (LEDs have a relatively long lifetime) and, because the majority of parts of the arrangement are sealed, the arrangement is resistant to damage from the environment. In an embodiment, the entire assembly fits within a laryngoscope handle. The assembly can therefore be removed if it fails and replaced with a replacement assembly. Advantageously, in an embodiment, the entire sealed "assembly" can be manufactured relatively inexpensively.

In an embodiment, the light source is activated by a magnetically operated reed switch. The switch is embedded within the device and no parts of the switch protrude from the laryngoscope body. The absence of switching components protruding from the laryngoscope body improves ease of sterilisation of the laryngoscope body and reduces opportunity for bacteria to become harboured in cracks or crevices in the laryngoscope body.

In accordance with a second aspect, the present invention provides a laryngoscope handle mounting a light apparatus in accordance with the first aspect of the present invention.

The laryngoscope blade or handle or both may be disposable.

In accordance with a third aspect, the present invention provides a laryngoscope mounting a light apparatus in accordance with the first aspect of the present invention.

The laryngoscope may be a disposable laryngoscope.

A light apparatus in accordance with the first aspect of the present invention may be utilised to retrofit an existing laryngoscope which was originally designed for a different type of light source.

In accordance with a fourth aspect, the present invention provides a method of retrofitting an existing laryngoscope, by mounting a light apparatus in accordance with the first aspect of the invention within the handle of the existing laryngoscope.

In accordance with a fifth aspect, the present invention provides a light source apparatus for a hand held medical apparatus, comprising a light source and electrical circuit for providing power to the light source and a remotely operable switch for switching power to the light source, characterised in that the remotely operable switch is arranged to switch the electrical circuit from an off state in which no current flows in the circuit to an on state in which current flows to power the light source.

Features and advantages of the present invention will become apparent from the following description of embodiments thereof, by way of example only, with reference to the accompanying drawings, in which:
Figure 1 is a diagram illustrating components of a light source apparatus in accordance with an embodiment of the present invention;
Figure 2 is a diagram showing the components of Figure 1 mounted within a sealed mounting; and
Figure 3 shows a top view of a laryngoscope blade;
Figure 4 is a cross sectional view of the blade of figure 3 along the line X-X;
Figure 5 is a perspective exploded view of a laryngoscope handle and blade;
Figure 6 is a detail view of the handle and blade of figure 5;
Figure 7 is a circuit diagram of a light source apparatus in accordance with a further embodiment of the present invention; and
Figure 8 is a diagram showing components of a light source apparatus in accordance with an embodiment of the present invention mounted within a handle of a laryngoscope.

Referring to the Figures, a light source apparatus in accordance with an embodiment of the present invention comprises an electrical circuit generally designated by reference numeral 1, and in this example being in the form of tracks 2 and contacts 3, 4 mounted on a printed circuit (PCB) 5. It also includes a light source 6, in this example being a light emitting diode (LED) 6. A remotely operable switch, in this example being a magnetically operable reed switch 8, is also included.

In this embodiment, a resistor 7 is included in the electrical circuit.

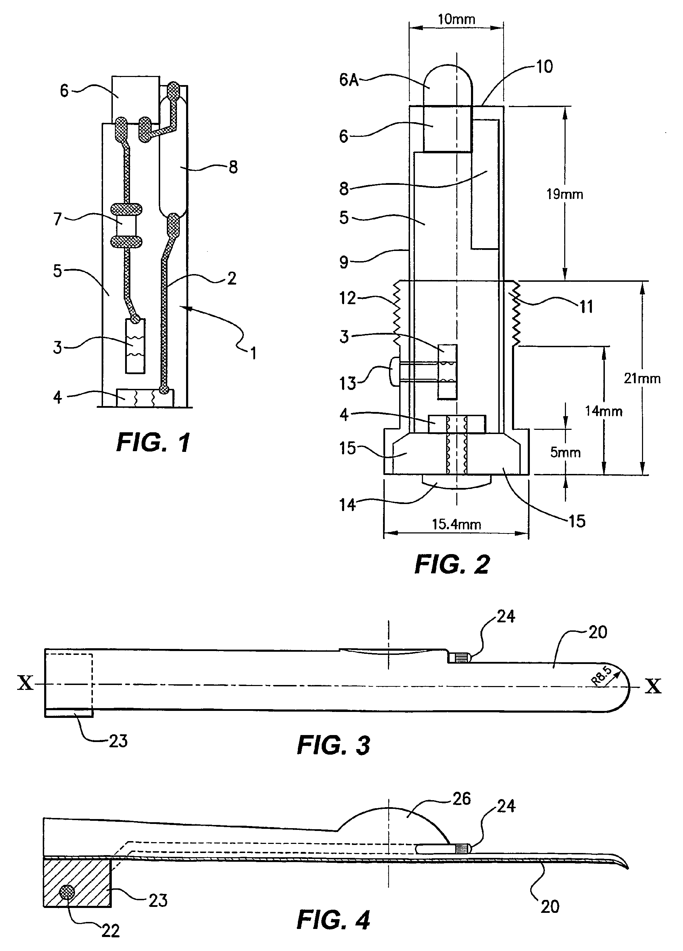

The PCB 5 illustrated in Figure 1 is mounted within a sealed mounting, in this embodiment being a resin potted cylinder 9 that permits passage of light from the LED. The potted polyester cylinder 9 seals the components from the environment. Referring to Figure 2, the LED 6 is ground down (6A) so that its top surface is flush with the top surface 10 of the potted cylinder 9.

The potted cylinder 9 is seated within a cylindrical metal body 11 (in this embodiment the metal is brass). The body 11 includes a thread 12 and the body 11 is arranged to fit into a laryngoscope handle. The threads 12 engage with corresponding threads on the inner surface of the laryngoscope handle. A case terminal screw 13 makes contact with the electrical contact 3 and a nipple terminal screw 14 makes contact with the electrical contact 4. A nylon insulator 15 seals the base of the metal body 11.

The entire assembly fits within a laryngoscope handle and the LED 6 optically communicates with an optical fibre extending in a laryngoscope blade. The co-operating blade incorporates a magnet in its proximal end, such that when the blade is fitted to the handle, the reed switch 8 is operated and power is provided to the LED 6. Batteries seated in the lower half of the handle provide the power source.

Referring to figure 3 and 4, a laryngoscope blade 20 is shown which includes a permanent magnet 22 housed in mounting block 23. An optical fibre bundle 24 in use guides light from LED 6 to be emitted towards the end of blade 20. The blade also includes soft palate guard 26.

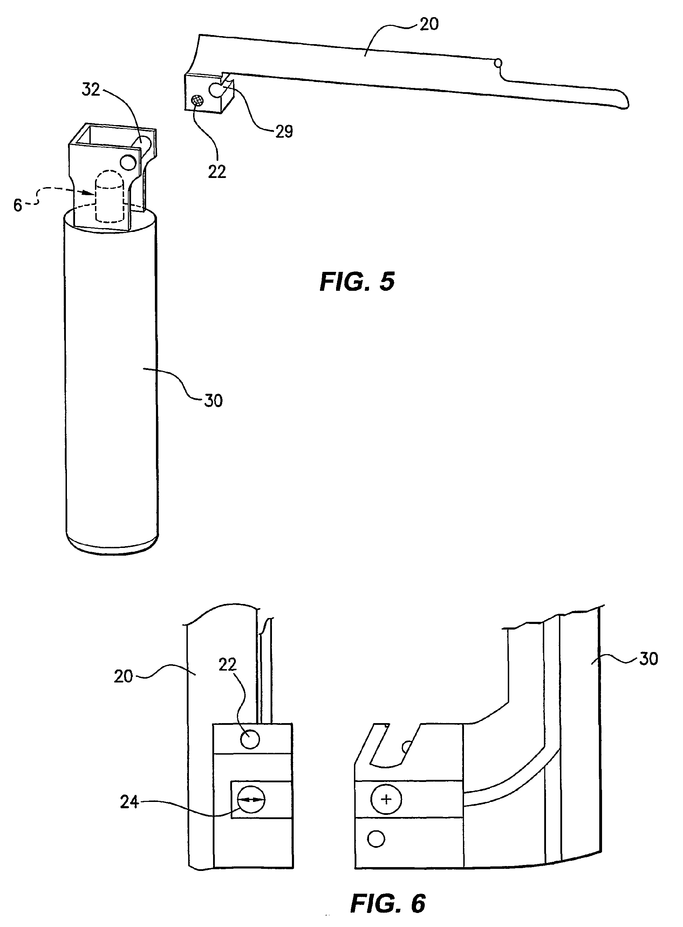

Referring to figures 5 and 6, blade 20 is shown alongside handle 30 in a disassembled state. Handle 30 includes a rod 32 to which handle can be rotatably mounted by a snap fit of rod 32 into channel 29. When mounted to the handle 30, blade can be rotated about rod 32 to extend the blade to an operational position. This brings magnet 22 into close proximity with reed switch 3 to active the reed switch. This causes illumination of LED 6. The light from LED 6 is guided into fibre optic bundle 24.

The sealed assembly may be utilised in any laryngoscope. In one embodiment the assembly is suitable for disposable laryngoscopes, as the assembly is relatively inexpensive.

In another embodiment, existing laryngoscopes may be retrofitted by adapting existing laryngoscope handles to receive the assembly and modifying blades to incorporate a magnet to activate the switch.

The assembly may also be used with laryngoscopes arranged for multiple use. The assembly may easily be replaced if the light source apparatus fails.

Because the assembly is sealed, it is substantially protected from the environment.

In the above embodiment, the remotely operable switch is a reed switch. Any other type of remotely operable switch may be used and the present invention is not limited to use of a reed switch.

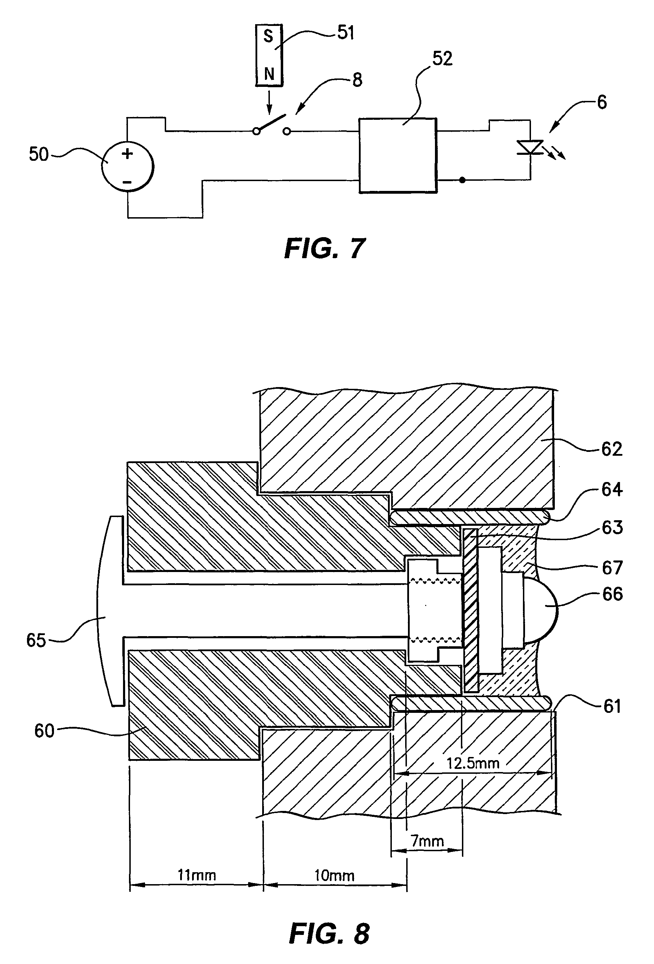

Figure 7 shows a circuit diagram for a further embodiment of the light source apparatus in accordance with the present invention. This circuit also includes a magnetically operable reed switch 8 and an LED 6. A power source (the battery in the laryngoscope handle) is designated by reference numeral 50. A diagram of a magnet 51 is shown to illustrate operation of the reed switch 8. In the described embodiment, magnet 51 corresponds to magnet 22 in the laryngoscope blade. In this embodiment, the circuit also includes a current or voltage driver 52 for providing constant spectral output and intensity of the LED 6.

Figure 8 is a diagram of a top part of a laryngoscope handle showing one way in which a light source assembly in accordance with an embodiment of the invention may be mounted within the laryngoscope handle. A delrin insert 60 is mounted proximate an opening 61 in the brass laryngoscope head 62. A PCB 63 mounting the components of the light source apparatus is mounted within the opening 61. Brass tube 64 surrounds the opening. A bolt 65 secures the light source apparatus. The LED 66 is mounted within the opening and a clear polyester resin 67 fills the opening, allowing 1 mm approximately of LED to be exposed. The opening 61 is therefore sealed off and the componentry within the opening is sealed from the environment.

Note that where dimensions are included, they are for the purposes of example only, and different embodiments of the invention may have different dimensions.

It can be seen that embodiments of the invention may have at least the following advantages:
- Longevity - LED cold light source
- Low power consumption
- Non-contact micro switch
- Durable - no moving parts
- Sealed electronics - waterproof
- Handle can be disinfected
- Cost effective - modular PCB construction

The light source apparatus of the present invention is suitable for use in devices other than laryngoscopes including hand held torches, medical auroscopes, ophthmascopes, vaginascopes and transilluminators.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

It will be appreciated by persons skilled in the art that numerous variations and/or modifications may be made to the invention as shown in the specific embodiments without departing from the scope of the invention as set out in the claims. The present embodiments are, therefore, to be considered in all respects as illustrative and not restrictive.

## Claims

1. A light source apparatus for a laryngoscope, including a light source (6), an electrical circuit (1) for providing power to the light source, a power source for providing power to the electrical circuit and a remotely operable switch (8) for switching power to the light source (6), wherein the remotely operable switch (8) is a magnetically activated switch (8) arranged to be operated by a magnet (22) mounted in a proximal part of a laryngoscope blade (20), and **characterized in that** it is arranged to switch the electrical circuit (1) from an off state in which no current flows in the circuit (1), to an on state in which current flows from the power source to the light source (6), to power the light source (6).

2. An apparatus in accordance with Claim 1, **characterised by** the light source apparatus being arranged to fit into a laryngoscope handle.

3. An apparatus in accordance with Claim 1, **characterised in that** the magnetically activated switch (8) is a reed switch (8).

4. An apparatus in accordance with any one of the preceding claims, **characterised in that** the light source (6) is a light emitting diode (LED) (6).

5. An apparatus in accordance with any one of the preceding claims, **characterised by** the light source apparatus being arranged to co-operate with an optical fibre (24) mounted in a laryngoscope blade (20), so that light is transmitted from the light source (6) to the optical fibre (24).

6. An apparatus in accordance with any one of the preceding claims, **characterised in that** the light source (6), switch (8) and electrical circuit (1) are mounted together in a sealed mounting (9, 11), forming a sealed assembly (9, 11) being arranged to fit into the handle of a laryngoscope.

7. An apparatus in accordance with any one of the preceding claims, **characterised in that** the light source (6) is current or voltage driven to provide constant spectral output and intensity.

8. A laryngoscope handle (30) mounting a light apparatus in accordance with any one of Claims 1 to 7.

9. A laryngoscope blade (20) and a light source apparatus in accordance with any one of Claims 1 to 7, wherein the blade (20) includes the magnet (22) for actuating the remotely operable switch (8) of the light source apparatus mounted in a proximal part of the laryngoscope blade (20).

10. A laryngoscope mounting a light apparatus in accordance with any one of Claims 1 to 7.

11. A laryngoscope in accordance with Claim 10, **characterised by** being a single use disposable laryngoscope.

12. A method of retrofitting an existing laryngoscope, **characterised by** mounting a light apparatus in accordance with any one of Claims 1 to 7 within the handle of the existing laryngoscope.

## Patentansprüche

1. Lichtquellengerät für einen Kehlkopfspiegel, das umfasst: eine Lichtquelle (6); einen elektrischen Stromkreis (1) für das Liefern von Strom zur Lichtquelle; eine Stromquelle für das Liefern von Strom zum elektrischen Stromkreis; und einen fernbetätigbaren Schalter (8) für das Schalten von Strom zur Lichtquelle (6), wobei der fernbetätigbare Schalter (8) ein magnetisch aktivierter Schalter (8) ist, der so angeordnet ist, dass er durch einen Magneten (22) betätigt wird, der in einem proximalen Teil einer Kehlkopfspiegelklinge (20) montiert ist, und **dadurch gekennzeichnet, dass** er angeordnet ist, um den elektrischen Stromkreis (1) von einem ausgeschalteten Zustand, in dem kein Strom im Stromkreis (1) fließt, in einen eingeschalteten Zustand zu schalten, in dem der Strom von der Stromquelle zur Lichtquelle (6) fließt, um die Lichtquelle (6) mit Strom zu versorgen.

2. Gerät nach Anspruch 1, **dadurch gekennzeichnet, dass** das Lichtquellengerät so angeordnet ist, dass es in einen Kehlkopfspiegelgriff passt.

3. Gerät nach Anspruch 1, **dadurch gekennzeichnet, dass** der magnetisch aktivierte Schalter (8) ein Reed-Schalter (8) ist.

4. Gerät nach einem der vorhergehenden Ansprüche, **dadurch gekennzeichnet, dass** die Lichtquelle (6) eine lichtemittierende Diode (LED) (6) ist.

5. Gerät nach einem der vorhergehenden Ansprüche, **dadurch gekennzeichnet, dass** das Lichtquellengerät angeordnet ist, um mit einer Lichtleitfaser (24) zusammenzuarbeiten, die in einer Kehlkopfspiegelklinge (20) montiert ist, so dass das Licht von der Lichtquelle (6) zur Lichtleitfaser (24) übertragen wird.

6. Gerät nach einem der vorhergehenden Ansprüche, **dadurch gekennzeichnet, dass** die Lichtquelle (6), der Schalter (8) und der elektrische Stromkreis (1) zusammen in einer abgedichteten Fassung (9, 11) montiert sind, die eine abgedichtete Baugruppe (9, 11) bildet, die so ausgebildet ist, dass sie in den Griff eines Kehlkopfspiegels passt.

7. Gerät nach einem der vorhergehenden Ansprüche, **dadurch gekennzeichnet, dass** die Lichtquelle (6) strom- oder spannungsgesteuert wird, um einen konstanten Spektralausgang und -intensität zu liefern.

8. Kehlkopfspiegelgriff (30), der ein Lichtgerät nach einem der Ansprüche 1 bis 7 aufnimmt.

9. Kehlkopfspiegelklinge (20) und ein Lichtquellengerät nach einem der Ansprüche 1 bis 7, bei dem die Klinge (20) den Magneten (22) für das Betätigen des fernbetätigbaren Schalters (8) des Lichtquellengerätes umfasst, der in einem proximalen Teil der Kehlkopfspiegelklinge (20) montiert ist.

10. Kehlkopfspiegel, der ein Lichtgerät nach einem der Ansprüche 1 bis 7 aufnimmt.

11. Kehlkopfspiegel nach Anspruch 10, **dadurch gekennzeichnet, dass** er ein wegwerfbarer Kehlkopfspiegel für einen einmaligen Gebrauch ist.

12. Verfahren zum Nachrüsten eines vorhandenen Kehlkopfspiegels, **gekennzeichnet durch** Montieren eines Lichtgerätes nach einem der Ansprüche 1 bis 7 innerhalb des Griffes des vorhandenen Kehlkopfspiegels.

## Revendications

1. Appareil source de lumière pour laryngoscope, comportant une source de lumière (6), un circuit électrique (1) destiné à fournir de l'énergie à la source de lumière, une source d'énergie destinée à fournir de l'énergie au circuit électrique et un commutateur actionnable à distance (8) destiné à commuter l'énergie vers la source de lumière (6), dans lequel le commutateur actionnable à distance (8) est un commutateur à activation magnétique (8) agencé pour être actionné par un aimant (22) installé dans une partie proximale d'une lame de laryngoscope (20) et **caractérisé en ce qu'**il est agencé pour faire passer le circuit électrique (1) d'un état d'arrêt dans lequel aucun courant ne circule dans le circuit (1) et un état de marche dans lequel du courant circule entre la source d'énergie et la source de lumière (6) afin de fournir de l'énergie à la source de lumière (6).

2. Appareil selon la revendication 1, **caractérisé en ce que** l'appareil source de lumière est agencé pour loger dans un manche de laryngoscope.

3. Appareil selon la revendication 1, **caractérisé en ce que** le commutateur à activation magnétique (8) est un interrupteur à lame (8).

4. Appareil selon l'une quelconque des revendications précédentes, **caractérisé en ce que** la source de lumière (6) est une diode électroluminescente (LED) (6).

5. Appareil selon l'une quelconque des revendications précédentes, **caractérisé en ce que** l'appareil source de lumière est agencé pour coopérer avec une fibre optique (24) installée dans une lame de laryngoscope (20) de manière que de la lumière soit transmise par la source de lumière (6) à la fibre optique (24).

6. Appareil selon l'une quelconque des revendications précédentes, **caractérisé en ce que** la source de lumière (6), le commutateur (8) et le circuit électrique (1) sont installés ensemble dans une monture étanche (9, 11), en formant un ensemble étanche (9, 11) agencé pour loger dans le manche d'un laryngoscope.

7. Appareil selon l'une quelconque des revendications précédentes, **caractérisé en ce que** la source de lumière (6) est pilotée en intensité ou en tension afin de fournir une production et une intensité spectrales constantes.

8. Manche de laryngoscope (30) dans lequel est installé un appareil source de lumière selon l'une quelconque des revendications 1 à 7.

9. Lame de laryngoscope (20) et appareil source de lumière selon l'une quelconque des revendications 1 à 7, dans laquelle la lame (20) comporte l'aimant (22) destiné à déclencher le commutateur actionnable à distance (8) de l'appareil source de lumière installé dans une partie proximale de la lame de laryngoscope (20).

10. Laryngoscope dans lequel est installé un appareil source de lumière selon l'une quelconque des revendications 1 à 7.

11. Laryngoscope selon la revendication 10, **caractérisé en ce qu'**il s'agit d'un laryngoscope à usage unique jetable.

12. Procédé de mise à niveau d'un laryngoscope existant, **caractérisé par** l'installation d'un appareil source de lumière selon l'une quelconque des revendications 1 à 7 dans le manche du laryngoscope existant.
